(19) Europäisches Patentamt
European Patent Office
Office européen des brevets

(11) EP 1 012 132 B1

(12) FASCICULE DE BREVET EUROPEEN

(45) Date de publication et mention de la délivrance du brevet:
**26.11.2003 Bulletin 2003/48**

(21) Numéro de dépôt: **98942799.2**

(22) Date de dépôt: **31.08.1998**

(51) Int Cl.[7]: **C07C 68/02**, C07C 69/96

(86) Numéro de dépôt international:
**PCT/FR98/01867**

(87) Numéro de publication internationale:
**WO 99/011597 (11.03.1999 Gazette 1999/10)**

# (54) PHOSGENATION SOUS PRESSION DES ALCOOLS POUR LA PRODUCTION DES CHLOROFORMIATES

DRUCKFOSGENIERUNG VON ALKOHOLEN ZUR HERSTELLUNG VON CHLOROFORMIATEN

PHOSGENATION UNDER PRESSURE OF ALCOHOL'S FOR PRODUCING CHLOROFORMATES

(84) Etats contractants désignés:
**DE FR GB**

(30) Priorité: **04.09.1997 FR 9711189**

(43) Date de publication de la demande:
**28.06.2000 Bulletin 2000/26**

(73) Titulaire: **SOCIETE NATIONALE DES POUDRES ET EXPLOSIFS**
**F-75181 Paris Cédex 04 (FR)**

(72) Inventeurs:
- **GAREL, Laurent**
  **F-69006 Lyon (FR)**
- **METZ, François**
  **F-69540 Irigny (FR)**

(56) Documents cités:
**EP-A- 0 542 132**
**DE-B- 1 213 419**
**FR-A- 2 484 406**

EP 1 012 132 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention a pour objet un procédé original pour accéder aux chloroformiates par phosgénation des alcools correspondants sous pression et sans catalyseur.

Les procédés classiques consistent à injecter du phosgène dans l'alcool seul ou en solution à pression ordinaire. On utilise généralement un excès de phosgène. Les évents constitués d'un mélange de phosgène et d'acide chlorhydrique sont non séparables à pression ordinaire, sauf à utiliser des condenseurs à très basse température, ce qui occasionne toujours une perte de phosgène.

La chimie est régie par l'équation réactionnelle suivante :

$$R(OH)n + nCOCl_2 \rightarrow R(OCOCl)n + nNCl \qquad (k)$$

Pour activer la réaction, il est nécessaire d'avoir recours à un ou plusieurs catalyseurs, d'où une littérature abondante pour ces dérivés. L'utilisation d'un catalyseur présente néanmoins plusieurs inconvénients. Leur coût tout d'abord puis leur influence sur le choix des matériaux car les catalyseurs rendent souvent le système réactionnel très corrosif. Ensuite, il favorise la formation de produits secondaires et le développement de coloration. Enfin, il implique une purification du chloroformiate par distillation ou cristallisation.

Dans la demande de brevet FR 2 484 406, il est décrit un procédé de préparation de chloroformiates facilement décomposables qui consiste à envoyer simultanément le phosgène et l'alcool dans une phase liquide de chloroformiate, sous une pression d'environ 0,1 atmosphère à 15 atmosphères, avec un rapport molaire du phosgène à l'alcool compris entre 1 et 2 et à une vitesse d'alimentation du phosgène et de l'alcool se situant de 30 % au dessous jusqu'à 30 % au-dessus de la vitesse de réaction du phosgène avec l'alcool. Mais ce procédé n'est utile que pour la fabrication des chloroformiates facilement décomposables. Le rapport phosgène à l'alcool est de préférence comprise entre 1,4 et 1,8 et la pression est de préférence de 1 atmosphère. Sa mise en oeuvre est complexe. Notamment elle implique le contrôle de la vitesse d'alimentation à la fois en phosgène et en alcool par rapport à leur vitesse de réaction. Sa durée est extrêmement longue.

L'invention cherche à éviter les inconvénients précités.

L'invention concerne un procédé de phosgénation d'alcools monohydroxy et/ou polyols pour accéder aux chloroformiates correspondants caractérisé en ce que l'on traite l'alcool et/ou le polyol, en présence ou non de solvant, par un excès molaire de phosgène, soit de 3 à 30 fois plus de phosgène par groupe hydroxyle, à une température comprise entre 0° et 200°C et à une pression comprise entre 2 et 60 bar (1 bar = $10^5$ Pa) sans catalyseur. On opère généralement en système fermé (pression autogène) ou en système ouvert (pression régulée par un dégazage partiel par exemple).

Le procédé est généralement mis en oeuvre en continu ou en semi continu. De préférence, on opère en système ouvert en faisant un dégazage partiel. Le dégazage doit se faire en veillant à ce que reste un excès de phosgène. Cela passe soit par l'élimination sélective de l'acide chlorhydrique, tout en gardant l'excès de phosgène et un peu de HCl soit par un dégazage incluant du phosgéne, ce dernier étant réalimenté en même temps. La température est avantageusement choisie entre 20 et 150°C, de préférence entre 25 et 80°C, alors que la pression est choisie de préférence entre 6 et 40 bar. Les conditions de température et pression sont déterminées par la nature de l'alcool et/ou du polyol et du chloroformiate correspondant, notamment le point critique et/ou le point de décomposition.

Les avantages de la phosgénation sous pression selon l'invention sont de pouvoir a) permettre d'éliminer les condenseurs à basse température, b) de se dispenser de catalyseur et c) d'obtenir les chloroformiates avec très peu ou pas de produits secondaires tels que des carbonates et des chlorures. Cela permet d'éviter la purification finale du chloroformiate obtenu, d'avoir une séparation simple en fin de réaction, de réduire le coût des utilités, et d'une façon générale les avantages sont ceux déjà discutés plus haut liés à l'absence de catalyseur.

Le procédé selon l'invention est avantageusement mis en oeuvre pour la conversion des alcools et/ou polyols de formule R(OH)n en chloroformiates R(OCOCI)n, n étant un entier de 1 à 6 et R étant défini comme :

- un radical aliphatique ayant de 1 à 22 atomes de carbone, linéaire ou ramifié, saturé ou non, éventuellement substitué a) par un ou plusieurs atomes d'halogène identiques ou différents, b) par un ou plusieurs group nitro ou c) par au moins un groupe alkyloxy, aryle (de préférence phényl), aryloxy ou arylthio, chacun de ces groupes étant non substitué ou substitué;
- un radical polyoxyalkyléne, linéaire ou ramifié, saturé ou non, éventuellement substitué par les substituants précédemment indiqués, de masse moléculaire comprise entre 200 et 6000 (les alcools devant être liquides ou pouvoir être dissous dans les conditions réactionnelles);
- un radical cycloaliphatique ayant de 3 à 8 atomes de carbone, portant ou non un ou plusieurs substituants choisis

parmi a) les atomes d'halogène, b) les radicaux alkyles ou haloalkyles, c) les groupes nitro et d) les radicaux aryles (de préférence phényl), aryloxy ou arylthio ceux-ci pouvant eux-mêmes être non substitués ou substitués;

- un radical carbocyclique aromatique, non substitué ou substitué par un ou plusieurs substituants choisis dans le groupe comprenant les atomes d'halogène, les radicaux alkyles ou haloalkyles (de préférence $CF_3$) ayant de 1 à 12 atomes de carbone (par exemple $C_2$-$C_6$ alkényl, $C_3$-$C_8$ cycloalkyl, $C_4$-$C_{10}$ cycloalkylalkyl, $C_7$-$C_{10}$ aralkyl, $C_7$-$C_{10}$ aralkoxy), les radicaux alkylthio ou haloalkylthio ayant de 1 à 6 atomes de carbone, les radicaux alkylsulfinyl ou haloalkylsulfinyl ayant de 1 à 6 atomes de carbone, les radicaux alkylsulfonyl ou haloalkylsulfonyl ayant de 1 à 6 atomes de carbone, les radicaux alkyloxy ou haloalkyloxy ayant de 1 à 6 atomes de carbone, les radicaux aryles, arylthio ou aryloxy, le groupe nitro;
- un radical hétérocyclique, aromatique ou non, à 5 ou 6 sommets, ayant un ou plusieurs hétéroatomes, identiques ou différents, choisis parmi les atomes d'oxygène, de soufre et d'azote et pouvant être non substitué ou substitué par un ou plusieurs substituants choisis parmi les atomes d'halogène, les groupes nitro, les radicaux alkyles, halogénoalkyles, alkyloxy, haloalkyloxy, aryles, arylthio, aryloxy, et/ou pouvant être éventuellement condensé avec un carbocyle aromatique, lui même non substitué ou substitué.

D'une manière générale. lorsqu'un groupe aryle (ou un de ses dérivés tels que aryloxy, arylthio) ou un carbocyle aromatique est mentionné, il faut considérer, même si ceci n'est pas dit au moment ou un tel radical apparaît afin d'alléger le présent exposé, que celui-ci peut porter des substituants choisis dans le groupe comprenant les atomes d'halogène, les radicaux alkyles, halogénoalkyles, alkyloxy, haloalkyloxy, alkylthio, haloalkylthio, alkylsulfinyl, haloalkylsulfinyl, alkylsulfonyl, haloalkylsulfonyl, aryles, aryloxy, arylthio, nitro.

Le procédé selon l'invention convient également pour la conversion de mélanges d'alcools monohydroxy et/ou mélanges d'alcools monohydroxy et polyols en chloroformiates correspondants.

Ce procédé selon l'invention est également caractérisé en ce que la pression est en outre utilisée pour faciliter la séparation de l'acide chlorhydrique et du phosgène, dans une colonne extérieure au réacteur, et ceci sans avoir recours à des condenseurs à basse température, source on l'a déjà vu de perte de $COCl_2$. La séparation devient dès lors plus simple, donc plus économique, qu'avec les procédés connus, et conduit à du phosgène facilement recyclable et à de l'acide chlorhydrique pur.

Les exemples suivant sont donnés à titre purement illustratifs de l'invention, qu'ils ne limitent en aucune façon.

**<u>Procédé de préparation de chloroformiates par phosgénation sous pression d'alcools monohydroxy.</u>**

Ces exemples montrent les avantages liés au procédé selon l'invention, procédé suivi par une méthode RMN à deux dimensions avec gradients de champ. Un suivi cinétique a également été effectué.

Les analyses RMN sous pression ont été effectuées sur un spectromètre AMX 300 opérant à 300 MHz pour le proton et équipé d'une sonde QNP $^1H/^{13}C/^{19}F/^{31}P$ gradient-z 5mm. Les déplacements chimiques (δ) des raies de résonance proton et carbone sont exprimés en ppm par rapport à du DMSO deutérié (39,5 ppm en RMN $^{13}C$ et 2,24 ppm en RMN $^1H$). Pour cet exemple, et pour les exemples qui suivent, le tube en saphir monocristallin a des diamètres externe et interne de 5 et 4 mm respectivement.

Dans ce tube, on introduit l'alcool et un capillaire contenant le DMSO deutérié pour assurer le verrouillage champ/fréquence. La tête en titane est adaptée sur le tube qui est alors immergé dans un bain de carboglace/acétone (-78°C) afin de condenser le phosgène (introduit par une vanne placée en haut de la tête du tube). On laisse revenir le milieu à tempéraure ambiante (environ 15 min) avant d'effectuer les mesures RMN.

<u>Exemple 1.</u> Phosgénation de l'alcool benzylique.

Dans le tube, on a introduit selon la procédure générale décrite ci-dessus 98,4 mg (0,89 mmol) d'alcool et 302,3 mg (3,05 mmol) de phosgène, soit un rapport molaire phosgène/alcool de 3,4. Le phosgène sert également de solvant.

Outre le phosgène, les trois composés suivants ont été caractérisés : l'alcool, le chloroformiate et le chlorure de benzyle.

Par contre, il ne s'est pas formé de carbonate de benzyle. On voit donc que, mis à part le fait que le chloroformiate est produit dans de bonnes conditions, les deux autres avantages de ce procédé résident dans la possibilité d'éviter ou limiter la production de carbonate et de chlorure.

Les pics RMN des protons $CH_2$ des trois produits précités sont bien séparés et identifiés. L'intensité de ces signaux caractéristiques est suivie au cours du temps. L'étude est menée à 300°K, et dès les premiers spectres, on remarque que le taux de transformation de l'alcool benzylique est important : supérieur à 90% (cf. tableau ci-dessous). Les résultats montrent qu'il se forme rapidement et majoritairement le chloroformiate.

| | | Proportions molaires % | |
|---|---|---|---|
| Temps (min) | Alcool benzylique | Chloroformiate | Chlorure de benzyle |
| 0* | 7 | 92 | 1 |
| 5 | 6 | 93 | 1 |
| 17 | 3 | 95 | 2 |
| 53 | 1 | 96 | 3 |
| 80 | < 1 | 97 | 3 |

* ce temps t=0 correspond à la fin de l'enregistrement du 1er spectre proton.

**Procédé de préparation de chloroformiates par phosgénation sous pression de diols.**

Les analyses RMN sous pression ont été effectuées sur un spectromètre AMX 300 opérant à 300 MHz pour le proton et équipé d'une sonde QNP $^1H/^{13}C/^{19}F/^{31}P$ gradient-z 5mm. Les déplacements chimiques (δ) des raies de résonance proton et carbone sont exprimés en ppm par rapport à du DMSO deutérié (39,5 ppm en RMN $^{13}C$ et 2.24 ppm en RMN $^1H$). Pour cet exemple, et pour les exemples qui suivent, le tube en saphir monocristallin a des diamètres externe et interne de 5 et 4 mm respectivement.

Dans ce tube, on introduit le polyol et un capillaire contenant le DMSO deutérié pour assurer le verrouillage champ/fréquence. La tête en titane est adaptée sur le tube qui est alors immergé dans un bain de carboglace/acétone (-78°C) afin de condenser le phosgène (introduit par une vanne placée en haut de la tête du tube). On laisse revenir le milieu à tempéraure ambiante (environ 15 min) avant d'effectuer les mesures RMN.

Exemple 2. Phosgénation sous pression de l'éthylène glycol.

Dans le tube, on a introduit selon la procédure générale décrite ci-dessus 105,1 mg (1,69 mmol) d'éthylène glycol et 1006 mg (10,2 mmol) de phosgène, soit un rapport molaire phosgène/glycol de 6. Le phosgène sert également de solvant.

La réaction à 300°K est suivie par RMN du proton et les produits de phosgénation sont identifiés par RMN du proton et du carbone à une et deux dimensions.

Après environ 20 minutes de réaction à 300°K (entre la fin de la préparation du tube et la première analyse RMN du proton), on observe 62 % molaire de dichloroformiate (1), 14 % molaire de carbonate (2) et 23 % molaire de produit (3) = ClCOO(CH2)2Cl. On n'observe pas d'éthylène glycol. La structure des produits formés est confirmée par une analyse Chromatographie Gazeuse/Infra-Rouge/Spectrométrie de Masse.

Les résultats montrent qu'il se forme rapidement et majoritairement le dichloroformiate.

| | | Proportions molaires % | |
|---|---|---|---|
| Temps (min) | (1) | (2) | (3) |
| 20 | 62 | 14 | 23 |
| 38 | 66 | 18 | 16 |
| 100 | 70 | 20 | 9 |
| 163 | 71 | 23 | 5 |
| 355 | 70 | 27 | 2 |

Exemple 3. Phosgénation sous pression d'un polyéthylène glycol (PEG).

Dans le tube, on a introduit selon la procédure générale décrite ci-dessus 113,8 mg (0.28 mmol) de polyéthylène glycol et 576,6 mg (5.82 mmol) de phosgène, soit un rapport molaire phosgène/PEG de 20,8. Le phosgène sert également de solvant.

Le polyéthylène glycol (PEG) choisi a une masse de 400 M environ.

Après environ 20 minutes de réaction à 300°K (entre la fin de la préparation du tube et la première analyse RMN du proton), on observe 4 massifs :

* 1 pic à 3,59 ppm correspondant aux protons $OCH_2$ "centraux"du PEG
* 1 massif à 3,71 ppm d'intensité 2
* 1 massif à 4,39 ppm d'intensité 2
* 1 massif large à 4,8 environ d'intensite 1

Ce spectre n'évolue pas à 300°K pendant 3 heures. Ensuite, nous avons chauffé à 373°K pendant 1 heure environ, nous avons toujours le même spectre.

Nous pouvons penser que nous avons :

* soit le produit (4) avec R' = OH probablement mais on ne peut exclure R'=Cl.

(4) 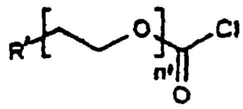 R'= OH, Cl

* soit un mélange équimolaire de PEG et de "dichloroformiate".

La première hypothèse semble la plus vraisemblable. La structure des produits formés ne peut pas être confirmée par une analyse Chromatographie Gazeuse/Infra-Rouge/Spectrométrie de Masse ; les composés ayant une masse trop élevée.

**Procédé de préparation de chloroformiates par phosgénation sous pression d'alcools aromatiques.**

Les analyses RMN sous pression ont été effectuées sur un spectromètre AMX 300 opérant à 300 MHz pour le proton et équipé d'une sonde QNP $^1H/^{13}C/^{19}F/^{31}P$ gradient-z 5mm. Les déplacements chimiques (δ) des raies de résonance proton et carbone sont exprimés en ppm par rapport à du DMSO deutérié (39,5 ppm en RMN $^{13}C$ et 2,24 ppm en RMN $^1H$). Pour cet exemple, et pour les exemples qui suivent, le tube en saphir monocristallin a des diamètres externe et interne de 5 et 4 mm respectivement.

Dans ce tube, on introduit l'alcool et un capillaire contenant le DMSO deutérié pour assurer le verrouillage champ/fréquence. La tête en titane est adaptée sur le tube qui est alors immergé dans un bain de carboglace/acétone (-78°C) afin de condenser le phosgène (introduit par une vanne placée en haut de la tête du tube). On laisse revenir le milieu à température ambiante (environ 15 min) avant d'effectuer les mesures RMN.

Exemple 4 Phosgénation sous pression du phénol.

Dans le tube, on a introduit selon la procédure générale décrite ci-dessus 43 mg (0,45 mmol) de phénol et 783 mg (7.9 mmol) de phosgène, soit un rapport molaire phosgène/phénol de 18. Le phosgène sert également de solvant.

Le tube saphir a été chauffé environ 10 h à 403°K (130°C) puis 7 h à 413°K (140°C) dans un bain d'huile silicone.

Après chauffage à 413°K, nous observons, en RMN du proton et du carbone, l'apparition de pics de faible intensité qui sont compatibles avec la présence de chloroformiate de phényle (les déplacements chimiques des raies de résonance proton et carbone 13 sont indiqués ci-dessous pour un produit de pureté molaire estimée supérieure à 98 %):

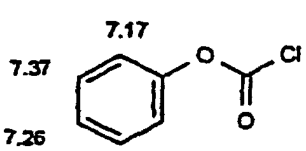

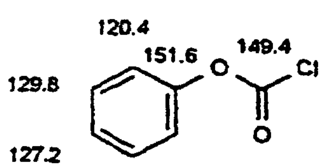

Exemple 5 Phosgénation sous pression du 2-naphtol.

Dans le tube, on a introduit selon la procédure générale décrite ci-dessus 31 mg (0,21 mmol) de 2-naphtol, 537 mg (5,4 mmol) de phosgène, soit un rapport molaire phosgène/naphtol de 26, et 688 mg (6,1 mmol) de chlorobenzène.
Le 2-naphtol introduit est entièrement soluble dans le chlorobenzène à 300°K. A 300°K, on n'observe aucune réaction du 2-naphtol. A 393°K, après 5 h, on observe l'apparition de signaux RMN de faible intensité que nous n'avons pas identifiés mais qui sont compatibles avec la présence de chloroformiate de 2-naphtyle.

**Procédé de préparation de chloroformiates par phosgénation sous pression en réacteur autoclave de 2 litres.**

Exemple 6. Préparation du chloroformiate de n-octyle.

Dans un réacteur autoclave de 2 litres, préalablement séché, équipé d'un condenseur alimenté avec de l'eau glycolée à -15°C et d'un système de régulation de pression, on introduit 890 g de monochlorobenzène, puis on ajoute 594 g (6 mol) de phosgène liquide en refroidissant le réacteur. On introduit ensuite rapidement 130 g (1 mol) de n-octanol et on ferme la vanne de mise à l'air. On chauffe le milieu réactionnel à 50°C. La pression relative atteinte est de 3,2 bars. On ajoute de l'argon pour obtenir une pression relative de 11,5 bars puis on maintient le mélange réactionnel à 50°C pendant 1 heure. La régulation de pression s'effectue à 11,2 bars relatifs sans nouvelle addition d'argon. Le milieu réactionnel est ensuite refroidi aux environ de 20°C et décomprimé.
L'analyse du milieu réactionnel par RMN 1H montre que le chloroformiate de n-octyle s'est formé et qu'il n'y a ni alcool résiduel, ni éther, ni carbonate. Par analyse en chromatographie en phase gazeuse, on détermine une teneur de 0,3% molaire en chloro-1-octane, soit une pureté estimée du chloroformiate de n-octyle de 99.7%.

Exemple 7. Préparation du chloroformiate d'un alcool lourd.

Dans le présent exemple, on phosgène l'alcool polyoxybutylène 1616 (POBA) de la société DOW CHEMICAL, de masse molaire moyenne 1400, de formule :

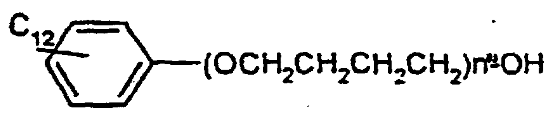

Dans le réacteur autoclave de 2 litres, équipé et séché comme à l'exemple 7, on charge le monochlorobenzène (500g) et on introduit 141 g de phosgène (1,42 moles) en refroidissant le milieu réactionnel à l'eau. On introduit alors par une pompe 200g d'alcool polyoxybutylène (0,143 moles) en solution dans 400 g de monochlorobenzène et on ferme la mise à l'air. On porte le milieu réactionnel à 60°C. La pression relative atteint 10 bars. On porte alors la pression relative à 11.5 bars par ajout d'argon sous pression. On maintient ces conditions pendant 4 heures. La régulation de pression relative s'effectue à 11.2 bars. On refroidit à température ambiante et la pression relative est alors de 8.8 bars, et on décomprime le milieu réactionnel. On obtient 1214 g pour une masse théorique de 1232,1 g.
Le suivi RMN n'est pas possible. Le rendement de la synthèse est déterminé par dosage du taux de phosgène et du taux de chlore hydrolysable sur une partie aliquote de milieu réactionnel.
Après dégazage et concentration. le taux de chlore hydrolysable est de 3,58% et le taux de phosgène est de 4,46% ce qui correspond à 0,126 moles de chloroformiate.
Le rendement en chloroformiate de l'alcool polyoxybutylène est de 88%.
Identification par Infra Rouge : Présence de bande CO à 1781 cm-1.
Le produit obtenu est de couleur comparable à celle de l'alcool de départ.

Exemple 8 Préparation du chloroformiate de perfluorohexyl-2-éthyle.

Dans le réacteur autoclave de 2 litres, traité et équipé comme précédemment, on introduit 600 mi de monochlorobenzene et 85 g de phosgène, en refroidissant le réacteur.
On ferme le réacteur, on le refroidit à 0°C et on introduit 50 g de perfluorohexyl-2-éthanol sous dépression. On ajoute de l'azote pour obtenir une pression absolue de 12 bars, puis, on chauffe le milieu réactionnel à 75°C, pendant 2 heures, tout en maintenant une régulation de pression.
On décomprime ensuite le réacteur et on distille le milieu réactionnel, sous pression réduite, pour éliminer le monochlorobenzene. On recueille 30,5 g de concentrat contenant en mélange 80 g de chloroformiate de perfluorohexyl-2-éthyle et 20 g de carbonate de diperfluorohexyl-2-éthyle.

## Revendications

1. Procédé de phosgénation d'alcools monohydroxy et/ou polyols pour obtenir les chloroformiates correspondants, **caractérisé en ce que** l'on traite l'alcool et/ou le polyol, en présence ou non de solvant, avec de 3 à 30 fois plus de phosgène en mole par rapport à chaque groupe hydroxyle, à une température comprise entre 0° et 200°C, à une pression comprise entre 2 et 60 bar et sans catalyseur.

2. Procédé selon la revendication 1, **caractérisé en ce que** l'on opère en système ouvert en faisant un dégazage partiel.

3. Procédé selon l'une des revendications 1 ou 2, **caractérisé en ce que** la température est comprise entre 20° et 150°C.

4. Procédé selon l'une quelconque des revendications précédentes, **caractérisé en ce que** la pression est comprise entre 6 et 40 bar.

5. Procédé selon l'une quelconque des revendications précédentes, **caractérisé en ce qu'**un alcool et/ou polyol de formule R(OH)n est converti en chloroformiate R(OCOCl)n, n étant un entier de 1 à 6 et R étant défini comme :

   - un radical aliphatique ayant de 1 à 22 atomes de carbone, linéaire ou ramifié, saturé ou non, éventuellement substitué a) par un ou plusieurs atomes d'halogène identiques ou différents, b) par un ou plusieurs groupes nitro ou c) par au moins un groupe alkyloxy, aryle, aryloxy ou arylthio, chacun de ces groupes étant non substitué ou substitué ;
   - un radical polyoxyalkylène, linéaire ou ramifié, saturé ou non, éventuellement substitué par les substituants précédemment indiqués, de masse moléculaire comprise entre 200 et 6000 (les alcools devant être liquides ou pouvoir être dissous dans les conditions réactionnelles) ;
   - un radical cycloaliphatique ayant de 3 à 8 atomes de carbone, portant ou non un ou plusieurs substituants choisis parmi a) les atomes d'halogène, b) les radicaux alkyles ou haloalkyles, c) les groupes nitro et d) les radicaux aryles, aryloxy ou arylthio ceux-ci pouvant eux-mêmes être non substitués ou substitués ;
   - un radical carbocyclique aromatique, non substitué ou substitué par un ou plusieurs substituants choisis dans le groupe comprenant les atomes d'halogène, les radicaux alkyles ou haloalkyles ayant de 1 à 12 atomes de carbone, C2-C6 alkényles, C3-C8 cycloalkyles, C4-C10 cycloalkylalkyles, C7-C10 aralkyles, C7-C10 aralkoxy, les radicaux alkylthio ou haloalkylthio ayant de 1 à 6 atomes de carbone, les radicaux alkylsulfinyle ou haloalkylsulfinyle ayant de 1 à 6 atomes de carbone, les radicaux alkylsulfonyle ou haloalkylsulfonyle ayant de 1 à 6 atomes de carbone, les radicaux alkyloxy ou haloalkyloxy ayant de 1 à 6 atomes de carbone, les radicaux aryles, arylthio ou aryloxy, le groupe nitro ;
   - un radical hétérocyclique, aromatique ou non, à 5 ou 6 sommets, ayant un ou plusieurs hétéroatomes, identiques ou différents, choisis parmi les atomes d'oxygène, de soufre et d'azote et pouvant être non substitué ou substitué par un ou plusieurs substituants choisis parmi les atomes d'halogène, les groupes nitro, les radicaux alkyles, halogénoalkyles, alkyloxy, haloalkyloxy, aryles, arylthio, aryloxy, et/ou pouvant être éventuellement condensé avec un carbocycle aromatique, lui même non substitué ou substitué.

6. Procédé selon l'une quelconque des revendications 1 à 4, **caractérisé en ce qu'**un mélange d'alcools monohydroxy est converti en chloroformiates.

7. Procédé selon l'une quelconque des revendications 1 à 4, **caractérisé en ce qu'**un mélange d'alcools monohydroxy et polyols est converti en chloroformiates.

## Patentansprüche

1. Verfahren zur Phosgenierung von einwertigen Alkoholen und/oder Polyolen zur Herstellung der entsprechenden Chlorformiate, **dadurch gekennzeichnet, dass** der Alkohol und/oder das Polyol in Gegenwart oder Abwesenheit eines Lösungsmittels mit der 3- bis 30-fachen Molmenge Phosgen, bezogen auf jede Hydroxygruppe, bei einer Temperatur im Bereich von 0 bis 200 °C und unter einem Druck von 2 bis 60 bar ohne Katalysator umgesetzt wird.

2. Verfahren nach Anspruch 1, **dadurch gekennzeichnet, dass** in einem offenen System unter teilweiser Entgasung gearbeitet wird.

3. Verfahren nach einem der Ansprüche 1 oder 2, **dadurch gekennzeichnet, dass** die Temperatur im Bereich von 20 bis 150 °C liegt.

4. Verfahren nach einem der vorhergehenden Ansprüche, **dadurch gekennzeichnet, dass** der Druck im Bereich von 6 bis 40 bar liegt.

5. Verfahren nach einem der vorhergehenden Ansprüche, **dadurch gekennzeichnet, dass** ein Alkohol und/oder Polyol der Formel R(OH)n in das Chlorformiat R(OCOCl)n umgewandelt wird, wobei n eine ganze Zahl im Bereich von 1 bis 6 bedeutet und R definiert ist als:

- eine geradkettige oder verzweigte, gesättigte oder ungesättigte aliphatische Gruppe mit 1 bis 22 Kohlenstoffatomen, die gegebenenfalls a) mit einem oder mehreren, gleichen oder verschiedenen Halogenatomen, b) mit einer oder mehreren Nitrogruppen oder c) mit mindestens einer Gruppe Alkyloxy, Aryl, Aryloxy oder Arylthio substituiert ist, wobei jede dieser Gruppen unsubstituiert vorliegt oder substituiert ist;
- eine geradkettige oder verzweigte, gesättigte oder ungesättigte Polyoxyalkylengruppe, die gegebenenfalls mit den oben angegebenen Substituenten substituiert ist, mit einer Molmasse im Bereich von 200 bis 6000 (wobei die Alkohole flüssig sein oder unter den Reaktionsbedingungen gelöst werden müssen);
- eine cycloaliphatische Gruppe mit 3 bis 8 Kohlenstoffatomen, die gegebenenfalls einen oder mehrere Substituenten trägt, die ausgewählt sind unter: a) Halogenatomen, b) Alkyl- oder Haloalkylgruppen, c) Nitrogruppen und d) Arylgruppen, Aryloxygruppen oder Arylthiogruppen, die selbst wiederum substituiert oder unsubstituiert vorliegen können;
- eine aromatische carbocyclische Gruppe, die unsubstituiert vorliegt oder mit einem oder mehreren Substituenten substituiert ist, die unter den Halogenatomen, Alkyl- oder Haloalkylgruppen mit 1 bis 12 Kohlenstoffatomen, $C_{2-6}$-Alkenylgruppen, $C_{3-8}$-Cycloalkylgruppen, $C_{4-10}$-Cycloalkylalkylgruppen, $C_{7-10}$-Aralkylgruppen, $C_{7-10}$-Aralkoxygruppen, Alkylthio- oder Haloalkylthiogruppen mit 1 bis 6 Kohlenstoffatomen, Alkylsulfinyl- oder Haloalkylsulfinylgruppen mit 1 bis 6 Kohlenstoffatomen, Alkylsulfonyl- oder Haloalkylsulfonylgruppen mit 1 bis 6 Kohlenstoffatomen, Alkyloxy- oder Haloalkyloxygruppen mit 1 bis 6 Kohlenstoffatomen, Arylgruppen, Arylthiogruppen oder Aryloxygruppen oder der Nitrogruppe ausgewählt sind;
- eine aromatische oder nicht aromatische, 5- oder 6-gliedrige heterocyclische Gruppe, die ein oder mehrere, gleiche oder verschiedene Heteroatome enthält, die unter Sauerstoff, Schwefel und Stickstoff ausgewählt sind, und die unsubstituiert vorliegt oder mit einem oder mehreren Substituenten substituiert sein kann, die unter den Halogenatomen, Nitrogruppen, Alkyl, Halogenalkyl, Alkyloxy, Haloalkyloxy, Aryl, Arylthio, Aryloxy ausgewählt sind, und/oder gegebenenfalls mit einem aromatischen Carbocyclus kondensiert sein kann, der selbst substituiert oder unsubstituiert vorliegt.

6. Verfahren nach einem der Ansprüche 1 bis 4, **dadurch gekennzeichnet, dass** ein Gemisch von einwertigen Alkoholen in die Chlorformiate umgewandelt wird.

7. Verfahren nach einem der Ansprüche 1 bis 4, **dadurch gekennzeichnet, dass** ein Gemisch von einwertigen Alkoholen und Polyolen in die Chlorformiate umgewandelt wird.

## Claims

1. Method for phosgenating monohydroxy alcohols and/or polyols so as to obtain the corresponding chloroformates, **characterized in that** the alcohol and/or polyol is treated in the presence or not of a solvent with 3 to 30 times more phosgene in mole in relation to each hydroxyl group, at a temperature of between 0° and 200°C, at a pressure of between 2 and 60 bar and without a catalyst.

2. Method according to claim 1, **characterized in that** an open system is used while performing partial degassing.

3. Method according to either of claims 1 or 2, **characterized in that** the temperature lies between 20° and 150°C.

4. Method according to any one of the preceding claims, **characterized in that** the pressure lies between 6 and 40 bar.

**5.** Method according to any one of the preceding claims, **characterized in that** an alcohol and/or polyol of the formula R(OH)n is converted into a chloroformate (R(OCOCl)n, n being an integer from 1 to 6 and R being defined as:

- a saturated or unsaturated linear or branched aliphatic radical having 1 to 22 carbon atoms, optionally substituted a) by one or more identical or different halogen atoms, b) by one or more nitro groups or c) by at least one alkyloxy, aryl, aryloxy or arylthio group, each of these groups being unsubstituted or substituted;

- a saturated or unsaturated linear or branched polyoxyalkylene radical, optionally substituted by the previously indicated substituents, with a molecular mass of between 200 and 6000 (the alcohols having to be liquid or capable of being dissolved under the reaction conditions;

- a cycloaliphatic radical having 3 to 8 carbon atoms, carrying or not one or more substituents chosen from among a) halogen atoms, b) alkyl or haloalkyl radicals, c) nitro groups and d) aryl, aryloxy or arylthio radicals, it being possible for these to be themselves unsubstituted or substituted;

- an aromatic carbocyclic radical, unsubstituted or substituted by one or more substituents chosen from the group comprising halogen atoms, alkyl or haloalkyl radicals having 1 to 12 carbon atoms, C2-C6 alkenyls, C3-C8 cycloalkyls, C4-C10 cycloalkylalkyls, C7-C10 aralkyls, C7-C10 aralkoxy, alkylthio or haloalkylthio radicals having 1 to 6 carbon atoms, alkylsulfinyl or haloalkylsulfinyl radicals having 1 to 6 carbon atoms, alkylsulfonyl or haloalkylsulfonyl radicals having 1 to 6 carbon atoms, the alkyloxy or haloalkyloxy radicals having 1 to 6 carbon atoms, aryl, arylthio or aryloxy radicals, the nitro group;

- an aromatic or non-aromatic heterocyclic radical with 5 or 6 apices, having one or more identical or different heteroatoms, chosen from among hydrogen atoms, sulfur and nitrogen, and that can be unsubstituted or substituted by one or more substituents chosen from among halogen atoms, nitro groups, alkyl, halogenoalkyl, alkyloxy, haloalkyloxy, aryl, arylthio, aryloxy radicals, and/or that can be optionally condensed with an aromatic carbo-ring that can itself be unsubstituted or substituted.

**6.** Method according to any one of claims 1 to 4, **characterized in that** a mixture of monohydroxy alcohols is converted into chloroformates.

**7.** Method according to any one of claims 1 to 4, **characterized in that** a mixture of monohydroxy alcohols and polyols is converted in chloroformates.

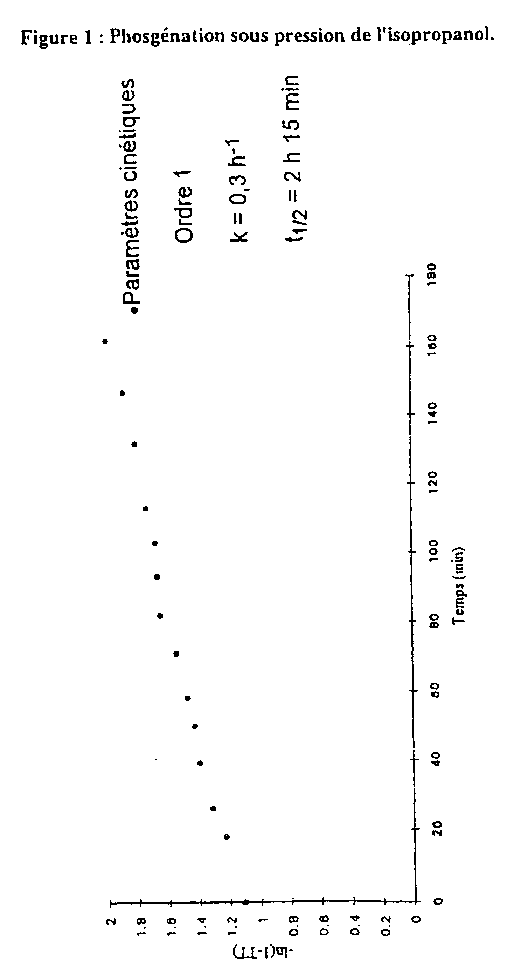

Figure 1 : Phosgénation sous pression de l'isopropanol.